# DEMANDE DE BREVET EUROPEEN

(11) **EP 1 038 859 A1**
(43) Date de publication de la demande: **27.09.2000**
(21) Numéro de dépôt: 00400400.8
(22) Date de dépôt: 11.02.2000
(51) Int. Cl.: C07C 29/141, C07C 29/145

(54) **Procédé continu de préparation d'un ose hydrogéné de haute pureté par hydrogénation catalytique**

(30) Priorité: 17.02.1999 FR 9902136
(71) Demandeur: Roquette Frères, 62136 Lestrem (FR)
(72) Inventeur: Choque, Jean-Christophe, 59000 Lille (FR); Fleche, Guy, 59190 Hazebrouck (FR)
(74) Mandataire: Boulinguiez, Didier

(57) **Abrégé**

L'invention concerne un procédé continu de préparation d'un ose hydrogéné de haute pureté par hydrogénation catalytique en réacteurs à ruissellement de l'ose correspondant, caractérisé par le fait que l'on conduit l'hydrogénation dans une succession de lits fixes de catalyseur au ruthénium disposés en série qui comprend :
a) une première zone d'hydrogénation constituée d'au moins un lit fixe de catalyseur au ruthénium, et
b) une seconde zone d'hydrogénation constituée d'au moins un lit fixe de catalyseur au ruthénium comportant un agent promoteur.

## Description

La présente invention est relative à un procédé continu de préparation d'un ose hydrogéné de haute pureté par hydrogénation catalytique de l'ose correspondant en réacteurs à ruissellement.

Plus particulièrement, l'invention concerne un procédé continu de préparation d'un ose hydrogéné de haute pureté par hydrogénation catalytique de l'ose correspondant dans une succession de lits fixes de catalyseur au ruthénium disposés en série et en deux zones de réaction, où une première zone est constituée d'au moins un lit fixe de catalyseur au ruthénium, et une seconde zone est constituée d'au moins un lit fixe de catalyseur au ruthénium comportant un agent promoteur, le tout fonctionnant de manière à ce que l'on obtienne l'ose hydrogéné avec une pureté, un taux de conversion et une sélectivité élevés en sortie de ladite seconde zone d'hydrogénation.

On entend par " ose " un glucide à n atomes de carbone comportant n-1 fonctions alcooliques et une fonction carbonyle. Lorsque la fonction carbonyle est une fonction aldéhyde, l'ose est un aldose, et lorsque la fonction carbonyle est une fonction cétone, l'ose est un cétose.

Au sens de l'invention, l'ose a un squelette carboné comprenant n atomes de carbone, n étant compris entre 3 et 7, de préférence compris entre 4 et 6.

L'ose peut en outre faire partie de la série D ou de la série L.

L'ose peut notamment être choisi dans le groupe comprenant les aldotétroses tels l'érythrose et le thréose, les aldopentoses ou aldohexoses tels que le glucose , le mannose, le galactose, le ribose, l'arabinose, le xylose, le lyxose. De préférence, l'ose est choisi parmi les aldoses, en particulier le D-glucose.

Les procédés d'hydrogénation catalytique des oses sont classiquement des procédés discontinus dans lesquels un catalyseur d'hydrogénation pulvérulent constitué de nickel de Raney est employé en suspension dans la solution d'ose.

Cependant, ces procédés discontinus ont l'inconvénient de présenter une productivité très faible et nécessitent des réacteurs importants et coûteux.

En outre, bien que le catalyseur au nickel de Raney puisse être filtré et réutilisé, une bonne part doit être remplacée par du nouveau catalyseur.

Enfin, la consommation en énergie de tels procédés reste importante.

Pour résoudre ces inconvénients, on a proposé de réaliser des procédés continus d'hydrogénation, en utilisant une pluralité de réacteurs en cascades contenant des catalyseurs pulvérulents constitués de nickel de Raney en suspension.

Cependant, l'activation, la circulation et la séparation sélective desdits catalyseurs pulvérulents entraînent en général encore des consommations importantes de catalyseurs.

Il a été ensuite décrit des procédés continus en lits fixes de catalyseur nickel/cuivre sur support inerte. Cependant, il est nécessaire de travailler en conditions de pression élevée et à faible vitesse superficielle d'écoulement.

Les températures élevées également utilisées conduisent à des réactions d'isomérisation, de craquage et à la caramélisation des oses, si bien que dans le cas de l'hydrogénation du D-glucose, le sorbitol produit contient en particulier une quantité importante de mannitol.

Un des premiers procédés continus d'hydrogénation utilisant un ou plusieurs réacteurs comportant des catalyseurs au nickel en lits fixes disposés en série est décrit dans le brevet US 2.650.941.

Cependant, les conditions de température et de pression sont encore élevées, conditions pourtant nécessaires pour atteindre un taux de conversion du glucose en sorbitol de l'ordre de 98,8 %.

D'autres procédés ont été décrits, procédés qui permettent d'augmenter ce taux de conversion, mais la formation d'acides dérivés d'oses inhérente à l'utilisation du nickel est la cause principale de la désactivation dudit catalyseur et de la baisse de sélectivité en produits hydrogénés de la réaction.

C'est ainsi que dans ces conditions, de l'acide gluconique est produit dans le procédé d'hydrogénation du glucose, ce qui provoque la lixiviation du nickel métallique et surtout la contamination du sorbitol produit par le nickel.

Un certain nombre d'autres métaux du groupe VIII ont été testés comme catalyseurs de substitution, tels que le palladium, le ruthénium et le platine.

C'est ainsi qu'il a été montré que les catalyses au palladium, au ruthénium et au platine consomment moins de métal que la catalyse au nickel, pour une réaction d'hydrogénation réalisée dans des conditions de température et de pression similaires. Le ruthénium est le catalyseur le plus performant.

Il permet en effet des conversions plus efficaces, y compris à des températures inférieures à 160°C et des conditions de pression de l'ordre de 100 bars. Il est de plus remarquablement stable en conditions acides, et permet par exemple l'hydrogénation de l'acide gluconique produit lors de la réaction d'hydrogénation du glucose.

Le brevet US 2.868.847 décrit ainsi l'utilisation de catalyseurs au ruthénium supportés sur charbon, alumine, silice ou kieselgühr pour l'hydrogénation en procédé discontinu d'hydrates de carbone en polyols.

Cependant, si cette hydrogénation catalytique au ruthénium du glucose en sorbitol permet d'éviter à la fois les réactions de caramélisation et la production d'acide gluconique parasite, la productivité reste trop faible pour être économiquement satisfaisante.

Les brevets US 4.380.679, US 4.380.680 et US 4.487.980 décrivent des procédés continus d'hydrogénation utilisant des lits fixes de catalyseur au ruthénium supportés sur charbon, alumine ou titane dioxyde, respectivement, et permettent de conduire l'hydrogénation dans de bonnes conditions.

Cependant, si les taux de conversion atteignent 99 %, les sélectivités sont relativement basses et ne dépassent pas les 96 %.

Le brevet US 3.963.789 décrit la mise en oeuvre de lits de catalyseur au ruthénium disposés en série, mais ce procédé demande la réalisation de plusieurs cycles d'opération avec régénération acide du catalyseur et, en outre, conduit encore à la production importante de produits d'isomérisation, i.e. principalement du mannitol, avec le sorbitol.

Le brevet US 4.476.331, décrit également un procédé continu multi-étages, le premier étage étant constitué par un catalyseur au ruthénium en lits fixes, et le second étage par des catalyseurs au ruthénium sulfurés.

Cependant, cette configuration a pour objet la production de sucres alcools dans le premier étage et leur craquage dans le second. Il y est décrit par exemple l'hydrogénation d'hexoses en hexitols dans le premier étage, et le craquage desdits hexitols en sucres alcools à 3 ou 4 atomes de carbone dans le second étage, en utilisant par ailleurs des conditions de température plus élevées.

Les brevets EP 300.018, US 4.626.604 et EP 319.208 décrivent des procédés continus multi-étages d'hydrogénation en conditions adiabatiques et en utilisant des lits fixes de catalyseur du groupe VIII, qui permettent d'atteindre des degrés de conversion des aldéhydes en leurs produits hydrogénés correspondants de l'ordre de 100 %. Il est cependant nécessaire de réintroduire de l'hydrogène à l'entrée de chaque réacteur à ruissellement, seul ou avec de nouvelles quantités d'aldéhydes à hydrogéner. En outre ces aldéhydes sont des composés présentant de préférence de 7 à 17 atomes de carbone de type oxo-aldéhydes.

Il apparaît donc clairement dans l'état de la technique que les procédés continus d'hydrogénation des oses ne permettent pas d'obtenir leurs produits hydrogénés correspondants avec des pureté et sélectivité élevées pour un taux de conversion élevé.

Surtout, ces procédés rendent obligatoire la purification poussée de l'ose hydrogéné obtenu pour éliminer toutes traces de co-produits. Dans le cas de l'hydrogénation du D-glucose en sorbitol, il s'agira notamment du mannitol, de l'arabitol et de l'iditol.

Cette nécessité génère donc des coûts supplémentaires de purification.

L'invention a donc pour but de remédier à cette situation, et de proposer un moyen permettant de répondre mieux aux diverses contraintes de la pratique, et notamment d'atteindre un bon compromis entre pureté, taux de conversion et sélectivité.

La société Demanderesse a trouvé, après de nombreuses recherches, qu'un tel moyen pouvait consister en un procédé continu d'hydrogénation catalytique particulier en réacteurs à ruissellement.

De manière surprenante et inattendue, la société Demanderesse a trouvé que le choix d'un catalyseur au ruthénium particulier, i.e. comportant un agent promoteur, et dans des conditions particulières, i.e. dans la constitution de la seconde zone d'hydrogénation, permet d'obtenir un ose hydrogéné avec une haute pureté, un taux de conversion et une sélectivité élevés encore jamais simultanément atteints par les procédés décrits dans l'état de la technique.

En effet, tous les procédés continus d'hydrogénation catalytique d'oses en leurs produits hydrogénés correspondants recommandent plutôt d'utiliser les mêmes qualités de catalyseur dans tous les lits fixes de catalyseur mis en oeuvre, et de gérer au mieux les conditions d'hydrogénation qui doivent permettre d'atteindre des valeurs satisfaisantes de pureté du produit, de conversion et de sélectivité.

La société Demanderesse a donc eu le mérite de montrer que l'on peut obtenir d'excellents résultats en mettant en oeuvre une combinaison particulière de lits de catalyseur au ruthénium en série dans un procédé continu d'hydrogénation classique en réacteurs à ruissellement.

On entend par " réacteur à ruissellement ", un réacteur d'hydrogénation dans lequel une phase liquide contenant le composé à hydrogéner et une phase gaz s'écoulent, par exemple de manière co-courante, dans un lit fixe de particules de catalyseur où s'effectue la réaction d'hydrogénation. Les débits d'écoulement de ces deux phases sont réglées pour permettre au liquide de ruisseler sur les particules de catalyseur et assurer un meilleur contact entre les deux phases liquide et gazeuse d'une part et la phase solide du catalyseur d'autre part.

La présente invention a précisément pour objet un procédé continu de préparation d'un ose hydrogéné de haute pureté par hydrogénation catalytique en réacteurs à ruissellement de l'ose correspondant, caractérisé par le fait que l'on conduit l'hydrogénation dans une succession de lits fixes de catalyseur au ruthénium disposés en série qui comprend :
a) une première zone d'hydrogénation constituée d'au moins un lit fixe de catalyseur au ruthénium, et
b) une seconde zone d'hydrogénation constituée d'au moins un lit fixe de catalyseur au ruthénium comportant un agent promoteur.

Au sens de l'invention, on entend par " haute pureté " un degré de pureté tel que la teneur en ose hydrogéné obtenue soit au moins égale à 98,3 %, de préférence au moins égale à 98,5 %, exprimée en poids sec de l'ose hydrogéné obtenu par rapport au poids total d'ose hydrogéné ou non et des co-produits.

Dans la description et les revendications, tous les pourcentages sont donnés en poids sauf indication contraire ou clairement non applicable.

De manière remarquable, le procédé conforme à l'invention permet d'obtenir un ose hydrogéné avec une teneur en chacun des co-produits de la réaction d'hydrogénation catalytique qui ne dépasse pas une valeur de plus de 0,5 %, de préférence 0,3 %.

A titre d'exemple, l'hydrogénation catalytique du D-glucose en sorbitol dans le procédé conforme à l'invention permet de limiter la formation de chacun des principaux co-produits de la réaction d'hydrogénation, i.e. l'iditol, l'arabitol et le mannitol, à une valeur de l'ordre de 0,25 %.

Dans le procédé conforme à l'invention, on fait donc passer une solution d'ose à hydrogéner dans une première zone d'hydrogénation constituée par au moins un lit fixe de catalyseur au ruthénium. De préférence, on choisit un seul lit fixe de catalyseur au ruthénium placé dans un réacteur à ruissellement.

La solution d'ose partiellement hydrogénée est ensuite acheminée dans la seconde zone d'hydrogénation constituée par au moins un lit fixe de catalyseur au ruthénium comportant un agent promoteur. De préférence, on choisit un seul lit fixe de catalyseur dans ce second réacteur à ruissellement.

Les lits fixes de catalyseur sont composés d'un empilement compact de particules qui contiennent du ruthénium imprégné ou co-échangé sur un support inerte.

Ce support inerte peut être choisi dans le groupe constitué par du charbon actif, des zéolites, de l'alumino-silicate, du titane dioxide, et de préférence il est constitué de charbon actif.

Le rapport pondéral ruthénium / support inerte est établi à une valeur comprise entre 0,5 et 5 %, de préférence compris entre 1 et 2 %.

Dans la seconde zone d'hydrogénation, le ou les lits fixes de catalyseur sont constitués de ruthénium comportant un agent promoteur.

Cet agent promoteur est choisi dans le groupe constitué du titane, du molybdène, du platine et du chrome. De préférence, il est constitué du platine.

Le rapport pondéral agent promoteur / support inerte constituant le lit fixe de catalyseur est alors choisi à une valeur comprise entre 0,1 et 0,3 %, de préférence de l'ordre de 0,2 %.

La première zone d'hydrogénation est alimentée, dans le procédé conforme à l'invention, par une solution d'ose dont la matière sèche est généralement comprise entre 20 et 50 %, de préférence comprise entre 30 et 50 %.

Dans un mode préférentiel du procédé conforme à l'invention, l'ose est choisi dans le groupe constitué par l'érythrose, le thréose, le glucose , le mannose, le galactose, le ribose, l'arabinose, le xylose, le lyxose et tous leurs isomères de fonction cétone. De préférence, on choisit le D-glucose comme ose à hydrogéner, et la matière sèche de ladite solution est de l'ordre de 40 %.

Le débit d'alimentation des solutions d'ose à hydrogéner par lit de catalyseur, dans le procédé conforme à l'invention, est compris entre 0,3 et 2 kg/l/h, de préférence compris entre 1 et 1,5 kg/l/h, exprimé en kg de solution d'ose par litre de lit fixe de catalyseur et par heure.

A titre d'exemple, pour une solution de D-glucose ayant une matière sèche comprise entre 30 et 50 %, de préférence de l'ordre de 40 %, le débit d'alimentation peut être fixé à une valeur de l'ordre de 1,2 kg/l/h.

La vitesse superficielle d'écoulement de la solution d'ose à hydrogéner à l'intérieur desdits lits fixes de catalyseur est maintenue à une valeur comprise entre 0,02 et 0,2 cm/s environ, de préférence à une valeur de l'ordre de 0,1 cm/s.

Les conditions de température et de pression d'hydrogénation sont également contrôlées de manière à conduire l'hydrogénation de l'ose en son produit hydrogéné correspondant avec une pureté, un taux de conversion et une sélectivité élevés.

Les conditions de température sont ainsi contrôlées à des valeurs inférieures à 120°C, de préférence comprises entre 80 et 105°C.

Les conditions de pression en hydrogène sont également fixées à des valeurs d'au moins 50 bars, de préférence comprises entre 50 et 150 bars.

A titre d'exemple, pour l'hydrogénation du D-glucose en sorbitol, la température d'hydrogénation peut être de l'ordre de 98°C au terme de la première zone d'hydrogénation, et de l'ordre de 99°C au terme de la seconde zone d'hydrogénation. La pression est quant à elle maintenue à 100 bars, comme exemplifié ci-après.

La société Demanderesse a également montré que le débit en hydrogène, dans le procédé conforme à l'invention, doit être tel que la quantité d'hydrogène introduite est au moins cinq fois, de préférence au moins dix fois supérieure à la stoechiométrie de la réaction.

Dans un mode préférentiel conforme à l'invention, les conditions d'hydrogénation, i.e. le débit d'alimentation, la matière sèche de la solution d'ose à hydrogéner, les conditions de température et de pression d'hydrogénation, le débit en hydrogène, sont réglés de manière à ce que l'on obtienne une hydrogénation substantielle de l'ose en son produit hydrogéné au terme de la première zone d'hydrogénation.

Au sens de l'invention, on entend par " hydrogénation substantielle ", une hydrogénation qui permette d'atteindre un taux de conversion compris entre 70 et 95 % environ, de préférence entre 80 et 95 %.

Cette solution d'ose partiellement hydrogénée est ensuite introduite dans la seconde zone d'hydrogénation, et le maintien des conditions opératoires à l'identique de celles utilisées dans la première zone d'hydrogénation, à l'exception de la constitution du lit fixe de catalyseur au ruthénium, permet d'obtenir un ose hydrogéné de haute pureté, avec un taux de conversion élevé, par exemple d'au moins 99,85 %, et une sélectivité également élevée, par exemple d'au moins 98 %, de préférence d'au moins 99 %.

D'autres caractéristiques et avantages de l'invention apparaîtront à la lecture des exemples non limitatifs décrits ci-dessous.

### Exemple 1.

La réaction d'hydrogénation est effectuée dans deux réacteurs à ruissellement connectés en série avec 1 échangeur thermique intermédiaire.

Les deux réacteurs renferment chacun un unique lit fixe de catalyseur, respectivement au ruthénium et au ruthénium / platine, dans lequel on fait circuler l'hydrogène et la solution de D-glucose de manière co-courante du haut vers le bas desdits réacteurs. Aucun ajout d'hydrogène supplémentaire n'est nécessaire à l'entrée de la seconde zone d'hydrogénation.

Chaque lit fixe de catalyseur au ruthénium est constitué d'un empilement compact de grains de catalyseur cylindriques.

Chaque grain de catalyseur est composé de charbon actif de type NORIT RX08, sous la forme d'un cylindre de 0,8 mm de diamètre et de 1 à 5 mm de longueur, sur lequel les métaux nobles de ruthénium, dans le premier réacteur à ruissellement, de ruthénium / platine dans le second réacteur à ruissellement, sont dans les proportions respectives de 2,11 % ruthénium et de 1,15 % ruthénium / 0,2 % platine.

Chaque réacteur contient 250 1 de catalyseur, disposé en lit fixe de 30 cm de diamètre et 3,5 m de hauteur.

On alimente simultanément la première zone d'hydrogénation avec une solution de D-glucose commercial à 40 % de matière sèche à un débit de 1,2 kg/l/h et de l'hydrogène à raison de 15 kg/h.

La pression de fonctionnement est de 100 bars, et les températures de préchauffage sont adaptées afin d'obtenir, en sortie du premier réacteur, la température de 98°C, et en sortie du second réacteur, une température de 99°C.

Le taux de conversion du D-glucose en sorbitol est déterminé, après analyse des produits d'hydrogénation par chromatographie en phase gaz, à une valeur de 90,5 %.

Le sirop hydrogéné à la sortie du second réacteur présente la composition donnée dans le tableau I suivant.

**Tableau I**

| Sucre alcools produits en final | % sec/sec |
|---|---|
| Sorbitol | 98,9 |
| Mannitol | 0,25 |
| Iditol | 0,25 |
| Arabitol | 0,25 |

On obtient un sorbitol de haute pureté, i.e. de l'ordre de 98 ,9 %, avec une sélectivité et un taux de conversion particulièrement élevés.

La sélectivité de la réaction est en effet de 99,2 %. Il est notamment remarquable qu'aucun des principaux co-produits ne soit présent avec le sorbitol à une teneur supérieure à 0,3 %. En outre, aucune production significative de maltitol, d'isomaltitol et d'acide gluconique n'est déplorée.

Le taux de sucres réducteurs libres, dosé par la méthode SOMOGY est de 500 ppm, ce qui donne un taux de conversion de 99,95 %.

### Exemple 2.

Le tableau II suivant rassemble les résultats obtenus dans le cadre du procédé continu d'hydrogénation conforme à l'invention de l'exemple 1 en comparaison avec les résultats obtenus, dans les mêmes conditions opératoires d'hydrogénation, mais sans ajouter d'agent promoteur au ruthénium composant le lit fixe de catalyseur qui constitue le second réacteur à ruissellement.

**Tableau II**

| Première zone Seconde zone | 2,11 % Ru 2,11 % Ru | 2,11 % Ru 1,15 % Ru / 0,2 % Pt |
|---|---|---|
| Sorbitol (%) | 98,2 | 98,9 |
| Mannitol (%) | 0,4 | 0,25 |
| arabitol (%) | 0,3 | 0,25 |
| Iditol (%) | 0,5 | 0,25 |
| Taux de conversion (%) | 99,8 | 99,95 |
| Sélectivité (%) | 98,5 | 99,2 |

En absence de promoteur, les trois co-produits principaux de la réaction d'hydrogénation du D-glucose en sorbitol en lits fixes de catalyseur au ruthénium seul sont chacun à une teneur au moins égale à 0,3 %

Les résultats montrent clairement l'avantage technologique , en termes de pureté et de sélectivité à des conversions élevées, que constitue la mise en oeuvre du procédé continu d'hydrogénation catalytique conforme à l'invention.

## Revendications

1. Procédé continu de préparation d'un ose hydrogéné de haute pureté par hydrogénation en réacteurs à ruissellement de l'ose correspondant, caractérisé par le fait que l'on conduit l'hydrogénation dans une succession de lits fixes de catalyseur au ruthénium disposés en série qui comprend :
a) une première zone d'hydrogénation constituée d'au moins un lit fixe de catalyseur au ruthénium, et
b) une seconde zone d'hydrogénation constituée d'au moins un lit fixe de catalyseur au ruthénium comportant un agent promoteur.

2. Procédé selon la revendication 1, caractérisé par le fait que l'agent promoteur utilisé avec le catalyseur au ruthénium dans la seconde zone d'hydrogénation est choisi dans le groupe constitué du titane, du molybdène, du platine et du chrome, et de préférence est du platine.

3. Procédé selon l'une ou l'autre des revendications 1 et 2, caractérisé par le fait que l'ose est choisi dans le groupe constitué par l'érythrose, le thréose, le glucose , le mannose, le galactose, le ribose, l'arabinose, le xylose, le lyxose et tous leurs isomères de fonction cétone, et de préférence est du D-glucose.

4. Procédé selon l'une quelconque des revendications 1 à 3, caractérisé par le fait que l'alimentation de la première zone d'hydrogénation est réalisée avec une solution d'ose dont la matière sèche est comprise entre 20 et 50 %, de préférence comprise entre 30 et 50 %.

5. Procédé selon l'une quelconque des revendications 1 à 4, caractérisé par le fait que dans chacune des zones d'hydrogénation, le débit d'alimentation de la solution d'ose à hydrogéner est compris entre 0,3 et 2 kg/l/h, de préférence compris entre 1 et 1,5 kg/l/h.

6. Procédé selon l'une quelconque des revendications 1 à 5, caractérisé par le fait que la vitesse superficielle d'écoulement du liquide traversant les lits de catalyseur au ruthénium disposés en série dans les deux zones d'hydrogénation est comprise entre 0,02 et 0,2 cm/s, et de préférence est de l'ordre de 0,1 cm/s.

7. Procédé selon l'une quelconque des revendications 1 à 6, caractérisé par le fait que la température dans les deux zones d'hydrogénation est inférieure à 120°C, de préférence comprise entre 80 et 105°C, et que la pression en hydrogène dans les deux zones d'hydrogénation est d'au moins 50 bars, de préférence comprise entre 80 et 150 bars.

8. Procédé selon l'une quelconque des revendications 1 à 7, caractérisé par le fait que le débit en hydrogène doit être tel que la quantité d'hydrogène introduite est au moins cinq fois, de préférence au moins dix fois supérieure à la stoechiométrie de la réaction.

9. Utilisation du procédé selon l'une quelconque des revendication 1 à 8 pour la préparation de sorbitol présentant une pureté d'au moins 98,3 %, de préférence d'au moins 98,5 %.
